# EUROPEAN PATENT SPECIFICATION

(11) **EP 2 024 788 B1**
(45) Date of publication and mention of the grant of the patent: **20.06.2018**
(21) Application number: 07795448.5
(22) Date of filing: 29.05.2007
(51) Int. Cl.: G03B 42/04

(54) **ADJUSTABLE DENTAL X-RAY IMAGE MEDIA HOLDER**
EINSTELLBARER ZAHNÄRZTLICHER RÖNTGENBILD-MEDIENHALTER
PORTE-SUPPORT D'IMAGE DE RADIOGRAPHIE DENTAIRE RÉGLABLE

(30) Priority: 30.05.2006 US 809491 P
(43) Date of publication of application: 18.02.2009
(60) Divisional application: 17199785.1
(73) Proprietor: DENTSPLY SIRONA Inc., York, PA 17401-2991 (US)
(72) Inventor: STEWARD, Curtis, L., Sandwich, IL 60548 (US); MCDONOUGH, Paul, Woodstock, IL 60098 (US); HARTLAUB, Thaddeus, J., Cherry Valley, IL 61016 (US)
(74) Representative: Blodig, Wolfgang
(86) International application number: PCT/US2007/012660
(87) International publication number: WO 2007/142925

(56) References cited:
- WO-A-2004/013691
- US-A- 1 012 561

## Description

### Technical Field

The present invention is related to dental x-ray image media holders. More particularly the invention relates to a device for securing a dental x-ray image media including film, phosphor plates, digital sensors and the like, and holding it in place relative to the x-ray target during the x-ray procedure. Specifically the invention relates to a holder that can be selectively used to take a plurality of different x-rays using a plurality of different sizes, shapes or configurations of image media. The inventive device has a bite block moveably affixed to an image media backing plate. The backing plate may be provided with flexible straps to secure an image media to the backing plate.

### Background of the Invention

Dental professionals have employed x-ray imaging for many years. A traditional dental x-ray procedure includes exposing an x-ray film to x-ray energy after it has passed through the target site. The film is developed and an image of the target site is achieved. It has also long been known that in order to obtain a useful image, the dental x-ray film must be positioned relative to the target site in a predetermined and secure manner. Many numbers of x-ray film holders and positioning devices have been developed, including for example, that shown in U.S. Pat. No. 3,473,026 which is hereby incorporated by reference for background purposes.

More recently, many dental professionals have used digital x-ray sensors in place of traditional x-ray films. An example of such a sensor is shown for example in U.S. Pat. No. 6,652,141 which is hereby incorporated by reference for background disclosure of x-ray sensors. As with x-ray films, it is necessary for the x-ray sensor to be secured in a predetermined position during the x-ray imaging procedure. In a manner similar to the use of x-ray films, holding and positioning devices have been developed for x-ray sensors. Digital sensors often have attached electrical connection cords such that the digital sensor transfers data to a storage or display device such as a computer.

Another type of image media common in the dental industry is a phosphor imaging plate. The x-ray shot is stored onto the imaging plate which is later read by a scanning machine or the like and the data is transferred to a storage or display device, such as a computer.

These and other type of devices that receive dental x-rays for dental purposes are herein collectively referred to as dental x-ray imaging media, sensors, imagers or the like. Any such devices that are sensitive to such x-rays is within the scope of the invention. It will be appreciated from the above discussion that the different image media holders while all accomplishing similar purposes, that is, dental diagnostics and the like, all operate in different manners. It is also the fact that the image media themselves are different in shape, size and configuration. For example, traditional x-ray films are often manufactured inside an envelope before being used with a patient. Phosphor imaging plates are often very thin, not much thicker than a sheet of paper or two and are placed into a barrier envelope before being used in an x-ray procedure. Digital sensors tend to be fairly thick in respective comparison due to the internal energy sensing components required for such devices. It is envisioned that in the future, other type of dental imaging media will be developed using similar or perhaps completely different technologies. These all have at least some commonality in that they generally must fit within the oral cavity and they must be securely held in a desired location during the x-ray procedure.

Adding to the complexity of using different imaging media is that even within a common type of media different manufacturers often provide media products that while they accomplish the same task as other media, are of a different size, shape or configuration.

Of course, it is also known that different set-ups must often be used for taking an x-ray image of different parts of the oral cavity. For example, conventionally dental x-rays taken in the oral cavity include anterior vertical periapical, anterior horizontal periapical, posterior horizontal periapical, posterior vertical periapical, horizontal bite-wing, vertical bite-wing, left and right images and other similar x-ray positions.

It will be appreciated that given the large number of different imaging media of different sizes, shapes and configurations, and given that many different x-ray procedures may be required in the oral cavity which require varied positioning of the imaging media relative to the tooth or other target site, the imaging media holder will have a different configuration for each possible combination. This requires the dental practitioner to normally stock a large number of imaging media holders in order to be reasonably certain that a proper holder is available at any given time for an x-ray procedure. It takes time and effort to match holders to specific imaging media.

A need exists therefore for a universal dental x-ray imaging media holder that will securely affix different shapes, sizes and configurations of such imaging media. It would also be desirable if the same holder could be used to hold such different media in a selected location during an x-ray procedure and which can be used to take more than one type of x-ray by being positioned at different locations in the oral cavity. The present invention provides an adjustable holder that meets these desires.

Documents US 1012561 and WO 2004/013691 represent further relevant prior art.

### Summary of the Invention

In general, a dental x-ray image media holder comprises an image media backing plate adjustably affixed to a bite block. The backing plate has at least one and preferably a plurality of channels for receiving a post affixed to the bite block, such that the bite block can be selectively moved within the channels to orient the bite block in a predetermined position relative to the backing plate. The invention is carried out by the invention as hereinafter described and and defined by the appended claims.

### Brief Description of the Drawings

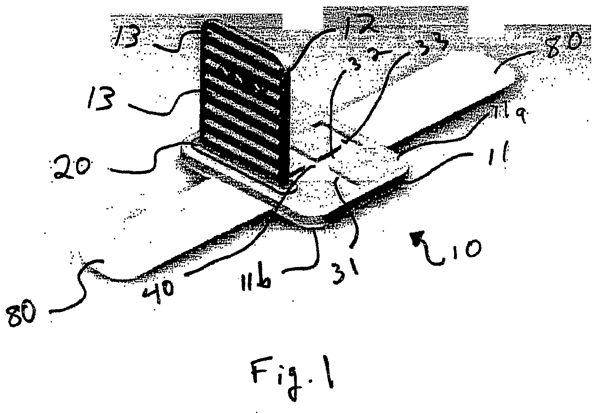

Fig. 1 is a perspective view of a dental image media holder embodying the concepts of the present invention.

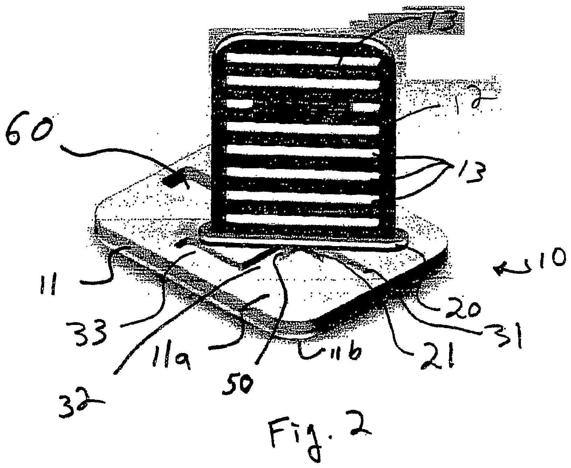

Fig. 2 is a perspective view of a portion of the image media holder of Fig. 1, showing a bite block in a position where adjustment thereof relative to a backing plate can take place.

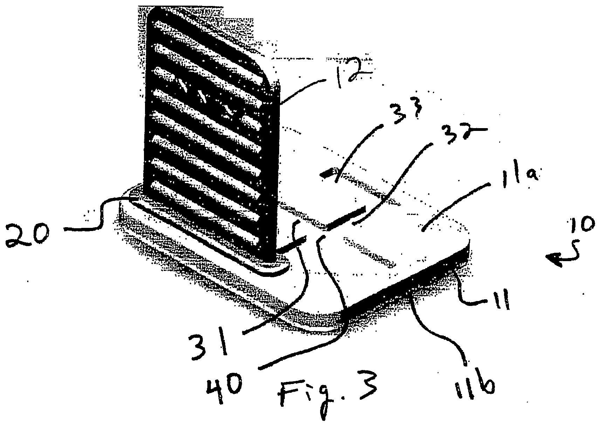

Fig. 3 shows the image media holder of Fig. 2 with the bite block in still another position.
Fig. 4 Fig. is a perspective view of a the image media holder as in Fig. 1, showing a bite block adjusted to a different position.
Fig. 5 is a partially exploded view of an image media holder as in Fig. 2 shown for environmental purposes in conjunction with a phosphor imaging plate and a barrier envelope for the phosphor plate.

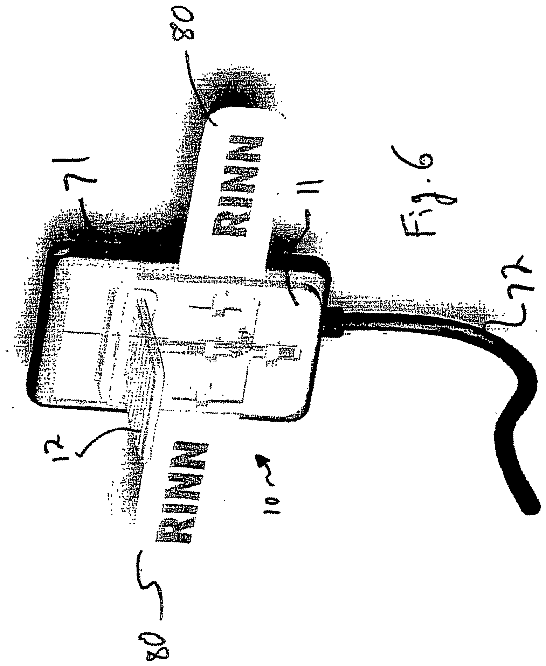

Fig. 6 is a top perspective view of an image media holder as in claim 1, shown for environmental purposes holding a digital dental sensor having an attached cord (partially shown).
Fig. 7 is a top perspective view of the image media holder of Fig. 6, showing a means of affixing an image media to the image media holder.
Fig. 8 is a bottom perspective view of the image media holder of Fig. 7.
Fig. 9 is a partially exploded view of the image media holder of Fig. 6, showing for further environmental purposes a cover sleeve for the image media.
Fig 10 is a perspective view of an alternative embodiment of an imaging media holder according to the present invention, and shown with an attached arm and aiming ring.
Fig. 11 is a top plan view of the holder of Fig. 10.
Fig. 12 is a front elevational view of the bite block component of the holder of Fig. 1.

### Preferred Embodiments for Carrying Out the Invention

An image media holder embodying the concepts of the present invention is generally designated on the attached drawings by the number 10. Image media holder 10 has a backing plate 11 configured to have a first side 11a and a second side 11b. While backing plate 11 may be of any size or shape, it has been found that a generally rectangular and flat plate is conducive to physically contacting and supporting a dental x-ray image media in a manner to be hereinafter described. Of course, any size, shape or configuration is within the scope of the invention although it will be exemplified and is preferred to be of the flat plate design as shown on the drawings.

Image media holder 11 is also provided with a bite block 12. It will be understood by those skilled in the art that a "bite block" used with a dental x-ray image media holder is intended to be physically impinged between the teeth, gums or other patient dentition during x-ray procedures. As is convention, the image media holder will be positioned in a patient's oral cavity (not shown) and the patient will be instructed to bite upon the block. This locates the affixed or supported x-ray image media during the ensuing procedure. Bite block 12 of the invention is of any suitable and conventional design, shape or configuration except for the unique inventive aspects to be described hereinbelow.

One preferred bite block 12 is generally flat and rectangular, and may be provided with gripping ridges 13 as is conventional. Also preferably, bite block 12 may be provided with a baseplate 20 at one end thereof, preferably at one.of the shorter ends of its rectangular body if a rectangular design is chosen. Baseplate 20 is configured to be generally smooth such that intimate physical contact between it and first side 11a of backing plate 11 can be made. Still more preferred, that physical contact is such that baseplate 20 can slide across the surface of first side 11a of backing plate 11 for purposes that will become clear from the following discussion.

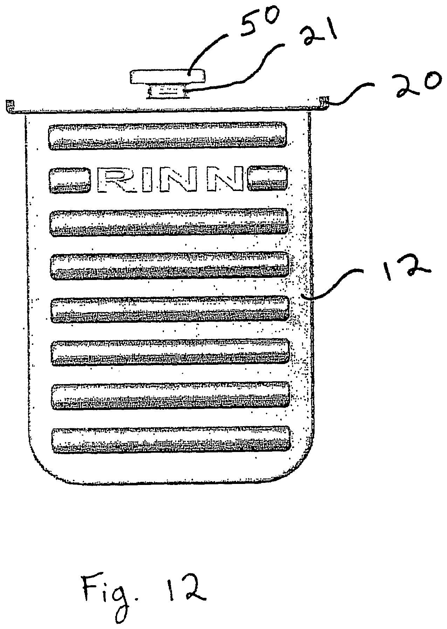

Extending from bite block 12 and more preferably from baseplate 20, is a positioning post 21 (Fig. 12) that will cooperatively interact with backing plate 11 in a manner to now be described.

In order to allow bite block 11 to be positioned in more than one position relative to backing plate 20, there is preferably provided in backing plate 11 at least one and preferably a plurality of slots or channels 31-33. Channels 31-33 may be of any design or configuration and may extend completely through backing plate 11 from first side 11a to second side 11b. Alternatively, channels 31-33 may extend only partially through from first side 11a and not all the way to second side 11b.

Post 21 affixed to bite block 12 is configured such that it can be received within channels 31-33. By sliding within a selected one of channels 31-33 to any preselected position therein, it will be appreciated that bite block 12 can be so positioned wherever it is desired. To allow positioning of bite block 12 relative to backing plate 11 and to accommodate at least the taking of dental x-rays to include anterior vertical periapical, anterior horizontal periapical, posterior horizontal periapical, posterior vertical periapical, horizontal bite-wing, vertical bite-wing or others, it is preferred to have a primary channel 31, a secondary channel 32, and a first and second tertiary channels 33, although of course, any number of channels of any design, shape or intersection is within the scope of the invention.

In one embodiment of the invention, channels 31-33 are substantially linear although any shape is within the scope of the invention. In this embodiment, primary channel 31 may intersect secondary channel 32 at some midpoint of the respective channels. By midpoint it is simply meant at some point between their respective ends. In a preferred configuration, the intersection 40 of primary channel 31 and secondary channel 32 is at the approximate center of secondary channel 32 and somewhat removed from the center of primary channel 31 such that the two from a "t". Tertiary channels 33 may intersect any other channels, but for the sake of the drawings a preferred embodiment is shown where each tertiary channel 33 at one of its own ends intersects an end of secondary channel 32.

As stated above, the exact design, dimensions or other characteristics of channels 31-33 can be varied but they should be such that they can receive and guide post 21 and hence, bite block 12. It will be appreciated that by sliding bite block 12 over the surface of backing plate 11, such sliding being guided by the physical contact of post 21 within a selected channel 31-33, bite block 12 can be positioned relative to backing plate 11 in any desired location. It will be further appreciated that bite block 12 can be moved from one channel such as primary channel 31 to another channel such as secondary channel 32 by moving post 21 (and hence the attached bite block 12) within the channel to the intersection thereof such as intersection 40 and thereby continue to move the post 21 to the other channel. Post 21 thus moves within a channel 31-33 in a sliding manner.

It is also preferred to provide a base 50 at an end of post 21 and provide channels 31-33 that completely pass through backing plate 11 between its two sides 11a and 11b, such that channels 31-33 are open slots. Post 21 thereby extends between and connects bite block 12 or baseplate 20 if employed, and base 50. By configuring base 50 to be wider than channels 31-33, and by configuring post 21 to be of suitable dimension, backing plate 11 can be caused to be physically received between base 50 and bite block 12. This physically affixes bite block 12 to backing plate 11 in an otherwise adjustable. manner by use of the channels 31-33 as already described. Again it will be appreciated that bite block 12 while being held to backing plate 11 is free to be positioned anywhere within channels 31-33 and may even be rotated on an axis or rotation provided by post 21, thereby accommodating any or all of the numerous x-ray positions required by a dental practitioner. In a preferred embodiment, the bite block 12 is rotated on post 21 with post 21 acting as an axle or rather its axis acts as an axis of rotation. In a further embodiment, post 21 and channels 31-33 are configured and dimensioned in shape or size such that bite block 12 can be rotated only at an intersection of at least two channels 31-33, although this is not necessarily required.

A larger opening or expanded area 60 may be provided in one or more channels, of such size as to pass base 50 to aid assembly of the image media holder 10 component parts. Expanded area may also provide an area where post 21 can be more easily rotated therein.

In a preferred embodiment of the invention, image media holder 10 is fabricated from any suitable material usable in the oral cavity. More preferred image media holder 10 is fabricated from a plastic material and the image media holder 10 is disposable.

There is also provided according to the invention some means of securing or affixing, preferably in a removable manner, an image media such as imaging plate 70 and digital sensor 71 to the second side 11b or backing plate 11. Two preferred methods include a releasable or pressure-sensitive adhesive (not shown) and straps 80, which may be used separately or in combination with each other.

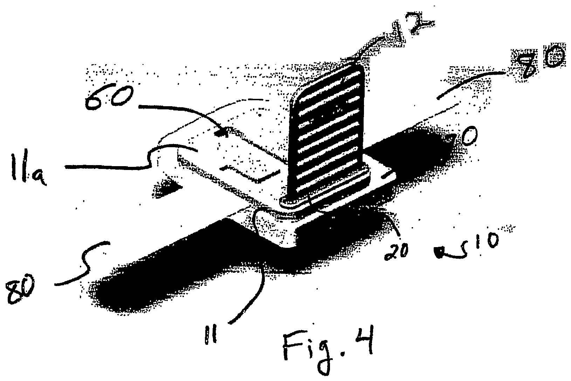

Any adhesive suitable for use in the oral cavity is within the scope of the invention, and the specific adhesive chosen is not necessarily a limitation of the invention. One preferred adhesive is a latex-free, pressure-sensitive adhesive which is coated onto second side 11b or backing plate 11 in any conventional manner. A release strip (not shown) may be employed to cover the adhesive until used. As shown for example, in Fig. 4, an image media such as plate 70 can be physically pressed onto second side 11b of backing plate 11 and held in place by the pressure-sensitive or other adhesive employed.

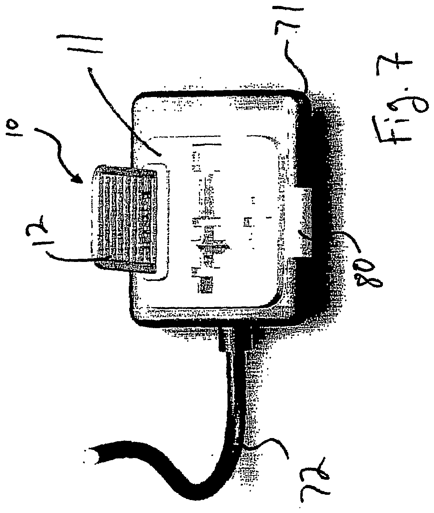

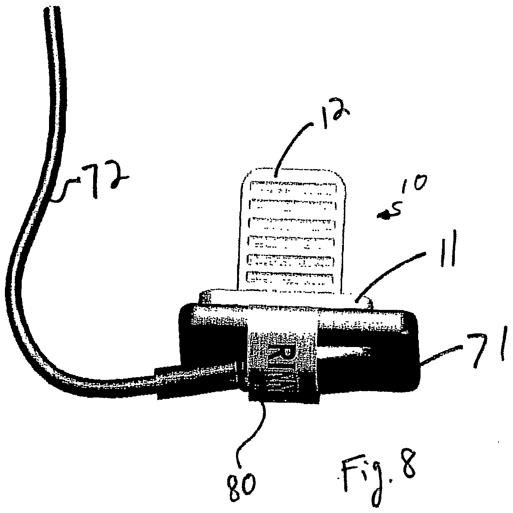

If a strap 80 is employed and two are preferred, it is affixed to backing plate 11 and is preferably flexible in one direction yet rigid in another. Once the image media such as plate 70 is caused to physically contact backing plate 11, strap 80 (or straps 80 if more than one are used) is wrapped around plate 70 (or any other imaging media as may be employed) in a manner allowed by the flexibility of strap 80, to thereby hold the imaging media to backing plate 11 (Figs. 7 and 8). Straps 80 may also be provided with a suitable adhesive or they may be provided with any other conventional means to affix them in the securing position. By all such manner or combinations thereof, straps 80 and/or the adhesive positively secure and otherwise affix (preferably in a removable manner) an imaging media to holder 10.

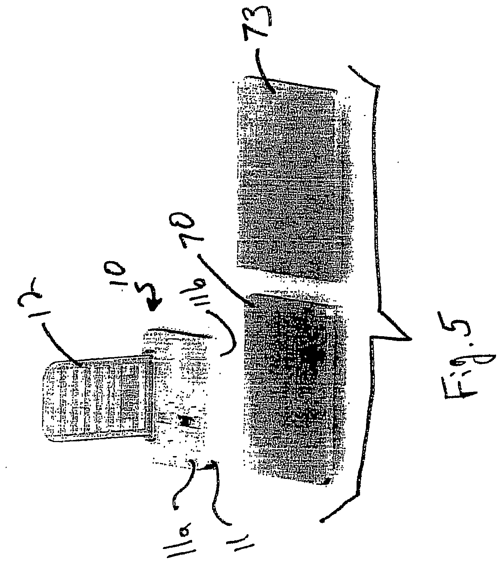

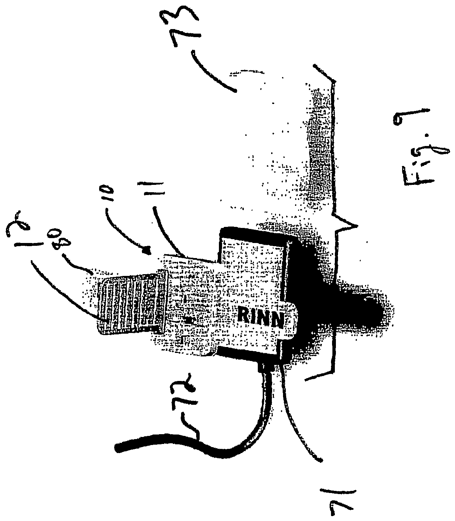

It will be appreciated that by suitably designing straps 80, any of a large number of image media designs such as for example, plate 70 or digital sensor 71 can be held by image media holder 10. Just as a belt may be adjusted to secure items of different size or shape, strap 80 may also accommodate different imaging media due to the length or other dimensions of strap 80. The design can accommodate different image media such as plate 70, relatively thicker image media such as digital sensor 71, and even other items such as connecting wire 72 for digital sensor 71 or conventional protective sleeves or barriers 73 (Figs. 5 and 9) for such image media. Although not depicted, a conventional dental x-ray film packet or indeed any other dental image media can be held by the inventive image media holder 10. It is even contemplated that the present invention can secure and hold such imaging media as may be developed in the future.

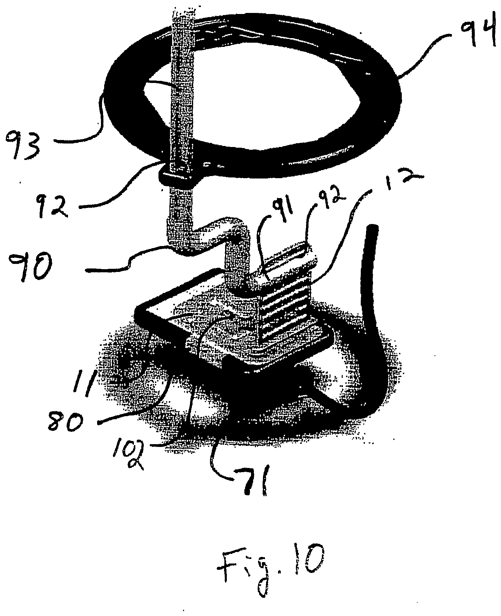

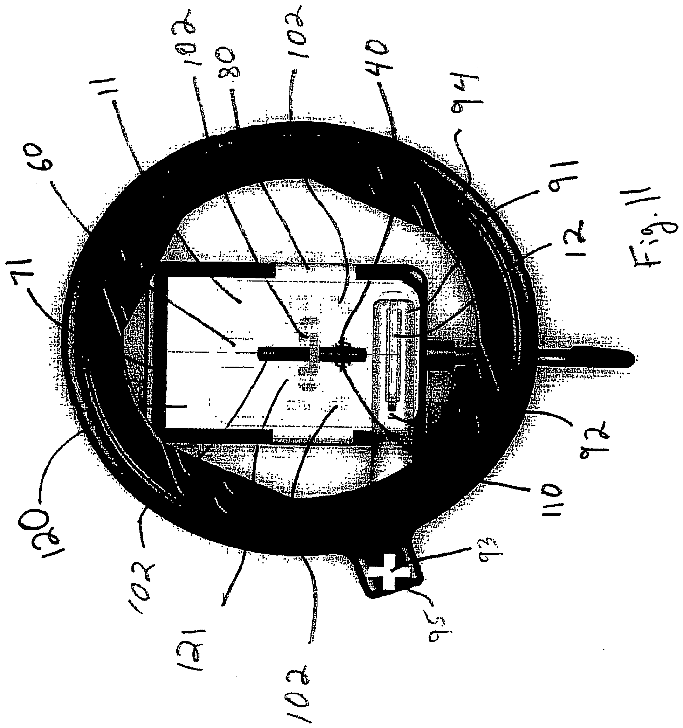

It is known in the dental x-ray art to provide aiming rings mounted upon arms or other structures to in turn, effectively connect the aiming ring to an imaging media holder. The present invention can be so configured as is shown in Figs. 10 and 11. Any means of affixing an arm and aiming ring to holder 10 is within the scope of the invention. One preferred means is to provide an arm 90 having a fixing member 91 provided with a slot 92. Slot 92 is configured and sized to physically receive and frictionally hold an edge of bite block 12 therein. At location distal to fixing member 91 is a support post 93 which is configured to receive or otherwise adjustably secure an aiming ring 94 thereto. For example, post 93 may have a certain shape such as the cross-shape depicted in the drawings and a complementary shaped aperture 95 may be provided at some location in aiming ring 94, such that post 93 is placed through aperture 95 in use. Aiming ring 94 is free to slide along post 93 to any desired position and is frictionally held in place by its physical contact with post 93.

In an alternative embodiment of the invention (Figs. 10 and 11), a backing plate 11 is provided with indicia 102 which separately indicate a different position for a specific x-ray procedure. Indicia 102 may be colors, numbers, letters, symbols, protrusions, detents, striations, or any other physical or visual indicators without limitation, or even combinations thereof. For example, backing plate 11 may be provided with more than one distinct indicia 102, which might indicate for example, by different colors the position to which bite block 12 should be moved to take a left bitewing or a right bitewing respectively. Further still, if holder 10 is of the type wherein bite block 100 can be rotated on post 21 as an axis of rotation and only at a certain point such as the intersection of certain channels 31-33, further indicia such as arrows 110 may be provided at the intersection or other area where such rotation is permitted.

Indicia 102 and 110 may be provided on a separate layer of material, such as a plastic sheet 120 which can be positioned in a juxtaposed physically contacting relationship with second side 11b of backing plate 11. In a preferred embodiment, straps 80 and integrally formed with and from the same material as sheet 120. In this configuration, it may be advantageous to provide a backing plate 11 which is at least partially transparent, such that indicia 102 placed upon sheet 120 may be viewed therethrough. While it will be appreciated that a transparent or at least partially transparent bite block 11 will facilitate viewing the indicia 102 or 110 therethrough when sheet 120 is employed, it is also possible to simply provide large enough openings 121 in bite block 100 so as to view indicia 102 or 110 therethrough. Further still, channels 31-33 may themselves be suitably positioned such that the indicia 102, 110 is viewable therethrough.

It will be appreciated therefore, that an image media holder 10 as described is capable of holding image media of different designs, shapes and configuration. The inventive image media holder is also capable of allowing the user to take dental x-rays in more than one position. All of these different uses can be accomplished with one image media holder according to the invention. The preferred embodiments for carrying out the invention have been described herein and shown on the attached drawings without attempting to show all variations that fall within the scope thereof. Therefore, the scope of the invention will be determined only by the attached claims.

## Claims

1. A dental x-ray image media holder (10) of the type having an image media backing plate (11) affixed to a bite block (12), the improvement comprising:
providing the backing plate (11) with at least one channel for receiving a post affixed to said bite block (12), such that the bite block (12) can be selectively moved within said channel to orient the bite block (12) in a predetermined position relative to the backing plate, and may be rotated on an axis of rotation provided by post (21),
**characterised in that**
the backing plate (11) is provided with at least two intersecting said channels (31, 32), whereby the bite block (12) can be moved from one channel (31) to another channel (32) by moving post (21), and hence the attached bite block (12), within the channel to an intersection thereof (40) and thereby continue to move the post (21) to the other channel (32).

2. A holder (10) as in claim 1, wherein said backing plate (11) is provided with at least four said channels, at least two of which intersect at midpoints thereof.

3. A holder (10) as in claim 2 wherein at least one of said four channels has a first and second end and intersects at least one other said channel at one of its said first or second ends.

4. A holder (10) as in claim 3, wherein said channels include a T-shaped configuration made up of a primary channel intersecting a secondary channel at a midpoint of each said primary and secondary channels, and at least one tertiary channel having a first and second ends and intersecting at its first end with an end of said secondary channel.

5. A holder (10) as in claim 1, wherein at least one of said channels is provided with an expanded area such that the bite block (12) can be rotated on said post relative to the backing plate.

6. A holder (10) as in claim 1, wherein said post has a base such that when positioned with said channel, a portion of said backing plate (11) is received between said base and the bite block (12) with said post extending between and connecting said base and the bite block.

7. A holder (10) as in claim 1 further comprising an aiming ring adjustably secured to the holder (10) with a support arm.

8. A holder (10) as in claim 7 wherein said support arm is affixed to said bite block.

9. A holder (10) as in claim 8 wherein said support arm is removably affixed to said bite block.

10. A holder (10) as in claim 9 wherein said support arm is provided with a slot for receiving said bite block, such that said bite block (12) is frictionally retained therein.

## Patentansprüche

1. Halter (10) für dentale Röntgenbildmedien des Typs mit einer an einem Beißblock (12) befestigten Bildmediumstützplatte (11), wobei die Verbesserung umfasst: das Versehen der Stützplatte (11) mit wenigstens einem Kanal zur Aufnahme eines am Beißblock (12) befestigten Pfostens, sodass der Beißblock (12) innerhalb des Kanals selektiv bewegt werden kann, um den Beißblock (12) in einer vorbestimmten Position relativ zur Stützplatte auszurichten, und auf einer durch den Pfosten (21) bereitgestellten Drehachse gedreht werden kann, **dadurch gekennzeichnet, dass** die Stützplatte (11) mit wenigstens zwei sich kreuzenden Kanälen (31, 32) ausgebildet ist, wodurch der Beißblock (12) von einem Kanal (31) zu einem anderen Kanal (32) bewegt werden kann, indem der Pfosten (21) und somit der angebrachte Beißblock (12) innerhalb des Kanals zu seinem Kreuzungspunkt (40) bewegt und dadurch fortgefahren wird, den Pfosten (21) zum anderen Kanal (32) zu bewegen.

2. Halter (10) nach Anspruch 1, wobei die Stützplatte (11) mit wenigstens vier der Kanäle ausgebildet ist, von denen sich mindestens zwei an ihren Mittelpunkten kreuzen.

3. Halter (10) nach Anspruch 2, wobei wenigstens einer der vier Kanäle ein erstes und ein zweites Ende aufweist und den wenigstens einen anderen Kanal an einem seines ersten oder zweiten Endes kreuzt.

4. Halter (10) nach Anspruch 3, wobei die Kanäle eine T-förmige Konfiguration einschließen, die aus einem primären Kanal, der einen sekundären Kanal an einem Mittelpunkt jeweils des primären und des sekundären Kanals kreuzt, und wenigstens einem tertiären Kanal mit einem ersten und einem zweiten Ende und der an seinem ersten Ende ein Ende des sekundären Kanals kreuzt, besteht.

5. Halter (10) nach Anspruch 1, wobei wenigstens einer der Kanäle mit einem erweiterten Bereich ausgebildet ist, sodass der Beißblock (12) auf dem Pfosten relativ zur Stützplatte gedreht werden kann.

6. Halter (10) nach Anspruch 1, wobei der Pfosten einen Sockel aufweist, sodass, wenn er im Kanal positioniert wird, ein Abschnitt der Stützplatte (11) zwischen dem Sockel und dem Beißblock (12) aufgenommen wird, wobei der Pfosten sich dazwischen erstreckt und den Sockel und den Beißblock verbindet.

7. Halter (10) nach Anspruch 1, weiterhin umfassend einen Visierring, der mittels eines Stützarms am Halter (10) einstellbar gesichert ist.

8. Halter (10) nach Anspruch 7, wobei der Stützarm am Beißblock befestigt ist.

9. Halter (10) nach Anspruch 8, wobei der Stützarm am Beißblock entfernbar befestigt ist.

10. Halter (10) nach Anspruch 9, wobei der Stützarm mit einem Schlitz zur Aufnahme des Beißblocks ausgebildet ist, sodass der Beißblock (12) darin reibschlüssig gehalten wird.

## Revendications

1. Monture de support d'image de radiographie dentaire (10) du type comportant une plaque d'appui de support d'image (11) fixée à un bloc porte-film (12), l'amélioration comprenant : la mise à disposition de la plaque d'appui (11) d'au moins un canal pour la réception d'un montant fixé audit bloc porte-film (12), de sorte que le bloc porte-film (12) puisse être déplacé de manière sélective dans ledit canal pour orienter le bloc porte-film (12) dans une position prédéterminée par rapport à la plaque d'appui, et puisse tourner sur un axe de rotation fourni par le montant (21), **caractérisé en ce que** la plaque d'appui (11) est pourvue d'au moins deux desdits canaux d'intersection (31, 32), permettant que le déplacement du bloc porte-film (12) soit possible d'un canal (31) vers un autre canal (32) en déplaçant le montant (21), et donc le bloc porte-film (12) monté dans le canal jusqu'à son intersection (40) et continuer ainsi à déplacer le montant (21) vers l'autre canal (32).

2. Monture (10) selon la revendication 1, dans lequel ladite plaque d'appui (11) est pourvue d'au moins quatre desdits canaux, dont au moins deux se croisent en leurs points médians.

3. Monture (10) selon la revendication 2, dans lequel au moins l'un desdits quatre canaux a une première et une seconde extrémité et intercepte au moins un autre dudit canal à l'une de ses première et seconde extrémités.

4. Monture (10) selon la revendication 3, dans lequel lesdits canaux comprennent une configuration en forme de T constituée d'un canal primaire croisant un canal secondaire en un point médian de chacun desdits canaux primaire et secondaire, et au moins un canal tertiaire ayant une première et une seconde extrémités et croisant au niveau de sa première extrémité une extrémité dudit canal secondaire.

5. Monture (10) selon la revendication 1, dans lequel au moins l'un desdits canaux est muni d'une zone dilatée de manière à ce que le bloc porte-film (12) puisse être tourné sur ledit montant par rapport à la plaque d'appui.

6. Monture (10) selon la revendication 1, dans lequel ledit montant dispose d'une base de sorte que, lorsqu'elle est positionnée avec ledit canal, une partie de ladite plaque d'appui (11) est logée entre ladite base et le bloc porte-film (12) avec ledit montant s'étendant entre et reliant ladite base et le bloc porte-film.

7. Monture (10) selon la revendication 1, comprenant en outre un cercle de visée fixé de manière réglable à la monture (10) avec un bras de support.

8. Monture (10) selon la revendication 7, dans lequel ledit bras de support est fixé audit bloc porte-film.

9. Monture (10) selon la revendication 8, dans lequel ledit bras de support est fixé de manière amovible audit bloc porte-film.

10. Monture (10) selon la revendication 9, dans lequel ledit bras de support est pourvu d'une fente pour la réception dudit bloc porte-film, de sorte que ledit bloc porte-film (12) est retenu par frottement à l'intérieur.
